# FASCICULE DE BREVET EUROPEEN

(11) **EP 3 651 708 B1**
(45) Date de publication et mention de la délivrance du brevet: **01.09.2021**
(21) Numéro de dépôt: 18749858.9
(22) Date de dépôt: 11.07.2018
(51) Int. Cl.: A61F 13/00, A61L 15/22, A61L 26/00

(54) **PANSEMENT INTERFACE**
SCHNITTSTELLENVERBAND
INTERFACE DRESSING

(30) Priorité: 12.07.2017 FR 1756599
(43) Date de publication de la demande: 20.05.2020
(73) Titulaire: Urgo Recherche Innovation et Développement, 21300 Chenove (FR)
(72) Inventeur: AUGUSTE, Stéphane, 21490 Ruffey les Echirey (FR); LAMOISE, Michel, 21110 Bessey les Citeaux (FR); DANEROL, Anne-Sophie, 21000 Dijon (FR); DOULIN, Erwann, 56400 Pluneret (FR)
(74) Mandataire: Plasseraud IP
(86) Numéro de dépôt international: PCT/FR2018/051745
(87) Numéro de publication internationale: WO 2019/012226

(56) Documents cités:
- EP-A1- 2 168 607
- WO-A1-87/05206
- WO-A1-2014/013175
- WO-A1-2015/092315

## Description

### RESUME

La présente invention est relative à un pansement interface.

Plus particulièrement, la présente invention se rapporte à un pansement interface comprenant une matrice élastomérique pourvue d'une pluralité de trous traversants.

### ETAT DE LA TECHNIQUE ANTERIEURE

On connaît depuis longtemps le traitement des plaies par des pansements destinés à être mis au contact d'une plaie en assurant une interface entre la plaie et une compresse absorbante que l'on dépose sur le pansement afin d'absorber des exsudats de la plaie. De tels pansements sont habituellement désignés par l'expression « pansements interface ».

Le pansement interface commercialisé depuis 2000 par la société Laboratoires URGO sous la dénomination URGOTUL® est un exemple illustratif de tels pansements interface.

Ce produit, notamment décrit dans l'exemple 1 de la demande de brevet WO 00/016725, est constitué d'une armature faite d'un tissu à mailles ouvertes dont les fils sont enrobés d'un gel cohésif de façon à laisser des trous traversants essentiellement non obturés et ayant chacun un contour carré autour d'un axe central. Le gel est formé d'une composition constituée d'une matrice élastomérique à base de copolymères tribloc du type ABA (styrène-oléfine saturée-styrène) fortement plastifiée et contenant en dispersion une faible quantité de particules hydrophiles d'un hydrocolloïde.

La composition qualitative et quantitative de la matrice élastomérique de ce pansement interface lui confère des propriétés remarquables en ce qui concerne la favorisation du processus de cicatrisation et, en particulier, de la prolifération des fibroblastes.

Le produit URGOTUL® présente néanmoins l'inconvénient, dans les cas où l'on souhaite l'appliquer sur des plaies difficilement recouvrables par exemple en raison de leur localisation, de manquer de conformabilité à cause de la rigidité de son armature.

Pour résoudre ce problème, des pansements interface autoportés, c'est-à-dire dépourvus d'armature, ont été décrits dans la demande de brevet FR 2 936 158. Les solutions proposées dans cette demande de brevet permettent d'obtenir des produits qui présentent une bonne élasticité, ainsi qu'une rigidité et une cohésion suffisantes pour pouvoir les manipuler.

Ces pansements interface posent néanmoins des difficultés de mise en œuvre pour les adapter à la partie du corps sur laquelle se trouve la plaie à recouvrir.

L'invention vise donc à améliorer la mise en œuvre des pansements interface, qu'ils soient autoportés ou non.

### RESUME DE L'INVENTION

A cet effet, l'invention propose un pansement interface comprenant une matrice élastomérique, la matrice élastomérique étant pourvue d'une pluralité de trous traversants, chaque trou traversant ayant un contour autour d'un axe central,
dans lequel au moins une partie de la pluralité de trous traversants est agencée de manière à former au moins un motif dans lequel chaque trou traversant est adjacent à au moins un autre trou traversant, les trous traversants adjacents ayant des contours dont des images par une translation dans un plan perpendiculaire aux axes centraux pour confondre des images desdits axes centraux par ladite translation ne se superposent pas.

Ces dispositions permettent d'obtenir un pansement interface pouvant être mis en forme, et en particulier découpé ou déchiré, de manière maîtrisée afin de l'adapter notamment aux dimensions et à la forme de la partie du corps sur laquelle se trouve la plaie à recouvrir. Le pansement interface pouvant être mis en forme de manière maîtrisée tant au niveau de bords extérieurs qu'au niveau d'ouvertures intérieures offre une mise en œuvre améliorée.

De plus, selon des dispositions particulières, le pansement interface selon l'invention soumis à une traction selon une direction parallèle à des bords latéraux garde ses bords latéraux droits. Ceci est particulièrement avantageux lors de l'application du pansement interface pour pouvoir l'étirer dans un sens, le sens d'une longueur par exemple, tout en conservant une même dimension dans un autre sens perpendiculaire, une largeur par exemple.

La matrice élastomérique peut être constituée d'une composition élastomérique, les trous traversants adjacents du motif étant séparés l'un de l'autre par un fil de composition élastomérique.

En particulier, le pansement interface peut être autoporté. Il est alors constitué de la seule matrice élastomérique et dépourvu d'armature supportant la composition élastomérique.

Ces dispositions permettent d'obtenir un pansement interface qui présente une bonne élasticité, une bonne flexibilité pour s'adapter à toutes parties du corps, y compris celles comportant des surfaces courbes avec un rayon de courbure faible, tout en présentant une certaine tenue lui assurant un maintien en place sur toutes parties du corps, y compris celles comportant des surfaces étendues avec un rayon de courbure faible.

La composition peut notamment comprendre:
- 5 à 20% en poids d'au moins un copolymère tribloc du type ABA, comportant deux blocs terminaux thermoplastiques A styrène et une séquence centrale élastomère B qui est une oléfine saturée ou d'un mélange de copolymères tribloc du type ABA, comportant deux blocs terminaux thermoplastiques A styrène et une séquence centrale élastomère B qui est une oléfine saturée, rapporté au poids total de la composition
- 50 à 80% en poids d'au moins un plastifiant,
- 5 à 20% en poids d'une résine,
les pourcentages étant rapportés au poids total de la composition.

Le motif peut comprendre des trous traversants adjacents ayant des contours identiques décalés l'un par rapport à l'autre angulairement autour des axes centraux respectifs. Ces dispositions permettent d'obtenir un motif selon lequel les trous traversants sont imbriqués les uns dans les autres.

En particulier, le motif peut comprendre des trous traversants adjacents ayant des contours dépourvus chacun de symétrie de rotation autour de l'axe central.

De façon complémentaire ou alternative, le motif peut comprendre des trous traversants adjacents ayant des contours présentant chacun une symétrie de rotation autour de l'axe central par rotation d'angle 2π/n radians, n entier, les contours des trous traversants adjacents du motif étant décalés l'un par rapport à l'autre angulairement autour des axes centraux respectifs avec des angles différents de 2π/n radians.

Le motif peut comprendre des trous traversants adjacents présentant des contours rectangulaires.

Les contours rectangulaires des trous traversants adjacents peuvent être agencés orthogonalement l'un par rapport à l'autre.

Le motif peut comprendre un réseau de trous traversants adjacents dont les axes centraux sont alignés selon au moins une première direction. Ces dispositions offrent une stabilité de forme dans la première direction.

Les axes centraux des trous traversants adjacents du réseau peuvent également être alignés selon une deuxième direction perpendiculaire à la première direction. Le pansement interface présente alors également une stabilité de forme dans la deuxième direction.

### DESCRIPTION DETAILLEE

D'autres objets et avantages de l'invention apparaîtront à la lecture de la description qui suit d'un mode de réalisation particulier de l'invention donné à titre d'exemple non limitatif, la description étant faite en référence aux dessins annexés dans lesquels :
- la figure 1 représente un pansement interface selon un premier exemple conforme à un mode de réalisation de l'invention, le pansement interface comprenant une matrice élastomérique pourvue de trous traversants, les trous traversants de contours rectangulaires identiques étant agencés de telle manière que deux trous traversants adjacents ont des contours décalés à 90° par rapport à des axes centraux respectifs,
- la figure 2 est une représentation d'un moule pour la fabrication du pansement interface selon le mode de réalisation de l'invention représenté sur la figure 1,
- la figure 3 est une représentation d'un moule pour la fabrication d'un pansement interface selon un deuxième exemple conforme à un mode de réalisation de l'état de la technique, les trous traversants ayant des contours carrés identiques,
- la figure 4 est une représentation du pansement interface selon le deuxième exemple conforme à l'état de la technique obtenu par moulage dans le moule de la figure 3,
- la figure 5 est une représentation d'un moule pour la fabrication d'un pansement interface selon un troisième exemple conforme à un mode de réalisation de l'état de la technique, les trous traversants ayant des contours carrés identiques de plus petites dimensions que ceux du deuxième exemple,
- la figure 6 est une représentation du pansement interface selon le premier exemple conforme à l'invention représenté sur la figure 1, le pansement interface ayant été découpé,
- la figure 7 est une représentation du pansement interface selon le deuxième exemple conforme à l'état de la technique représenté sur la figure 4, le pansement interface ayant été découpé,
- la figure 8 est une représentation du pansement interface selon le troisième exemple conforme à l'état de la technique obtenu à partir du moule représenté sur la figure 5, le pansement interface ayant été découpé,
- la figure 9 est une représentation du pansement interface selon le premier exemple conforme à l'invention représenté sur la figure 1, le pansement interface étant étiré dans une direction d'étirement,
- la figure 10 est une représentation d'un pansement interface selon un cinquième exemple conforme à un mode de réalisation de l'invention dans lequel le pansement interface est obtenu par impression en trois dimensions (3D), le pansement interface étant étiré dans la direction d'étirement,
- la figure 11 est une représentation du pansement interface selon un quatrième exemple dans lequel les trous traversants ont des contours en octaèdre identiques, le pansement interface étant étiré dans la direction d'étirement,
- la figure 12 est une représentation du pansement interface selon un sixième exemple conforme à un mode de réalisation de l'état de la technique, les trous traversants ayant des contours carrés identiques, le pansement interface étant étiré dans la direction d'étirement.

Sur les figures, les mêmes références désignent des éléments identiques ou analogues.

### Forme du pansement

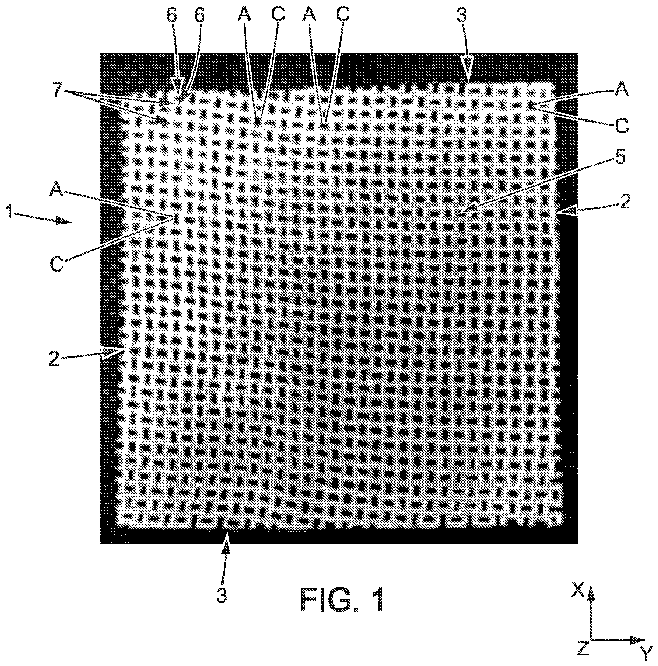

La figure 1 représente un pansement interface 1 destiné à être placé entre une plaie et une compresse absorbante de manière à pouvoir absorber les exsudats de la plaie tout en permettant le retrait de la compresse.

Le pansement interface 1 comprend une matrice élastomérique 5, élastique, souple et flexible tout en présentant une certaine tenue. Sans y être limité, le pansement interface 1 est de forme sensiblement carrée présentant deux bords latéraux 2 s'étendant selon une direction longitudinale X et deux bords transversaux 3 s'étendant selon une direction transversale Y, perpendiculaire à la direction longitudinale X, lorsqu'il est posé à plat sur une surface support. Le pansement interface 1 présente une épaisseur, mesurée selon une direction verticale Z perpendiculaire aux directions longitudinale X et transversale Y, faible par rapport à une longueur mesurée selon la direction longitudinale X et une largeur mesurée selon la direction transversale Y.

La matrice élastomérique 5 est constituée d'une composition élastomérique dont un mode de réalisation particulier sera décrit à titre purement illustratif plus en détails dans la suite de la description. Dans le mode de réalisation représenté, le pansement interface 1 est autoporté, c'est-à-dire qu'il est constitué de la seule matrice élastomérique 5 et est dépourvu d'armature supportant la composition élastomérique.

La matrice élastomérique 5 est pourvue de trous traversants 6. Les trous traversants 6 peuvent être répartis avec une densité telle qu'une surface totale des trous traversants représente entre 20 % et 75 % et, de préférence, entre 30 % et 65 % d'une surface totale du pansement interface 1.

Les trous traversants 6 sont agencés de manière à former un motif dans lequel chaque trou traversant 6 est adjacent à un ou plusieurs autres trous traversants 6. Dans le mode de réalisation particulier représenté, le motif comprend des trous traversants 6 répartis de façon régulière pour former un réseau de trous traversants 6 qui sont alignés selon une première direction, à savoir la direction longitudinale X, et selon une deuxième direction perpendiculaire à la première direction, à savoir la direction transversale Y. Chaque trou traversant 6 est alors adjacents à deux trous traversants 6 agencés de part et d'autre selon la direction longitudinale X ainsi qu'à deux trous traversants 6 agencés de part et d'autre selon la direction transversale Y. Les trous traversants 6 adjacents sont séparés deux à deux par un fil 7 de composition élastomérique.

Chaque trou traversant 6 a un contour C autour d'un axe central A. Selon l'invention, les trous traversants 6 adjacents ont des contours C dont des images par une translation dans un plan perpendiculaire aux axes centraux A pour confondre des images de leurs axes centraux A par cette translation ne se superposent pas.

En particulier, sur la figure 1, les contours des trous traversants 6 adjacents sont identiques et rectangulaires. Afin que leurs images par la translation précitée ne se superposent pas, les contours C des trous traversants 6 adjacents sont décalés les uns par rapport aux autres angulairement autour des axes centraux A respectifs. Pour ce faire, dans le mode de réalisation représenté, le contour C rectangulaire de chaque trou traversant 6 présente une symétrie de rotation autour de l'axe central A par rotation d'un angle de π radian, soit 180°. Les contours des trous traversants 6 adjacents du motif sont alors décalés l'un par rapport à l'autre angulairement autour des axes centraux A respectifs avec un angle différent de π radian et, par exemple, de π/2 radian, soit 90°. Chaque trou traversant 6 s'étendant dans l'une des directions longitudinale X et transversale Y est alors adjacent à quatre trous traversants 6 (deux selon la direction longitudinale X et deux selon la direction transversale Y) agencés orthogonalement, c'est-à-dire s'étendant dans l'autre des directions longitudinale X et transversale Y. Il en résulte une alternance de trous traversants 6 s'étendant longitudinalement et de trous traversants 6 s'étendant transversalement dans chacune des directions longitudinale X et transversale Y. En appliquant fictivement la translation dans un plan perpendiculaire aux axes centraux A pour amener l'axe central A de l'un des trous traversants 6 sur l'axe central A de l'un des trous traversants 6 adjacents, les images des contours ainsi obtenues ne se superposent pas.

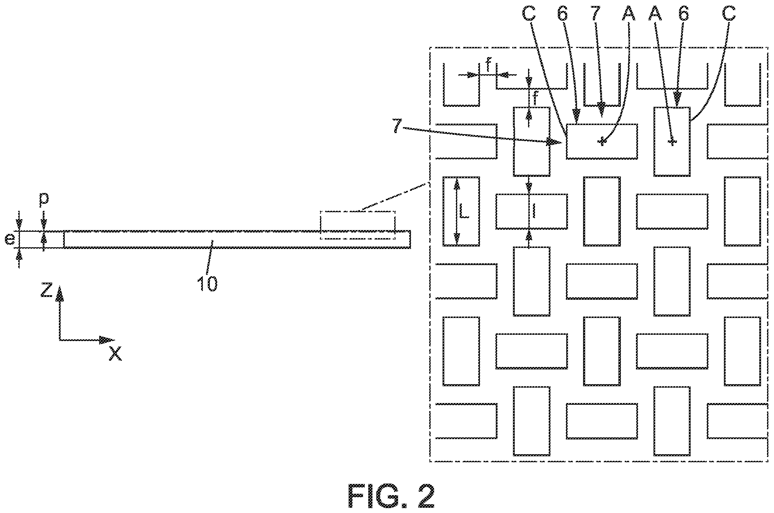

Comme représenté sur la figure 2, afin de réaliser le pansement interface 1, la matrice élastomérique 5 est formée en couche mince par coulage à chaud de la composition élastomérique sur une plaque 10 gravée avec le motif retenu pour former des trous traversants 6, suivi d'un refroidissement et d'un démoulage. La plaque 10 présente une épaisseur e, par exemple de l'ordre de 10 mm, et le motif présente une profondeur p adaptée pour obtenir l'épaisseur de la matrice élastomérique 5 souhaitée et, par exemple, comprise entre 0,4 mm et 2 mm, de préférence entre 0,5 mm et 1 mm, de préférence encore de l'ordre de 0,8 mm.

Alternativement, les trous traversants 6 peuvent être réalisés par perforation ou poinçonnage d'une composition élastomérique préalablement formée en couche mince, seule ou associée à un support provisoire ou à un film protecteur habituellement utilisé pour la fabrication de pansement.

Les trous traversants peuvent également être réalisés par une enduction tramée sur un support provisoire.

Le pansement interface peut encore être obtenu par impression en trois dimensions (3D).

Selon un premier exemple non-limitatif donné à titre purement illustratif, le pansement interface 1 se présente sous la forme d'un filet aéré (ou grille) obtenu par moulage et ayant :
- une épaisseur comprise entre 0,4 mm et 2 mm, de préférence entre 0,5 mm et 1 mm, de préférence encore de l'ordre de 0,8 mm,
- une largeur de fil f entre deux trous traversants 6 adjacents comprise entre 0,3 mm et 4 mm, de préférence entre 0,5 mm et 2 mm et par exemple de 0,8 mm,
- un grammage compris entre 200 g/m² et 1200 g/m², de préférence compris entre 300 g/m² et 800 g/m², par exemple de l'ordre de 390 g/m²,
- une longueur de trou traversant L comprise entre 2 mm et 4 mm, par exemple de l'ordre de 2,95 mm,
- une largeur de trou traversant 1 comprise entre 1 mm et 2 mm, par exemple de l'ordre de 1,45 mm.

Les trous traversants 6 ont alors une surface généralement comprise entre 0,25 mm² et 7 mm².

L'invention décrite en relation avec un mode de réalisation particulier dans lequel des trous traversants 6 de contour C rectangulaire sont régulièrement répartis n'est pas limitée à un tel motif.

La matrice élastomérique 5 pourrait notamment comprendre plusieurs motifs différents.

Dans d'autres modes de réalisation, le motif ou l'un des motifs pourrait comprendre des trous traversants 6 adjacents ayant des contours C présentant chacun une symétrie de rotation autour de l'axe central A par rotation d'angle 2π/n radians, n entier. Les contours C des trous traversants 6 adjacents du motif seraient alors décalés l'un par rapport à l'autre angulairement autour des axes centraux A respectifs avec des angles différents de 2π/n. Le motif ou l'un des motifs pourrait également comprendre des trous traversants 6 adjacents ayant des contours C dépourvus chacun de symétrie de rotation autour de l'axe central A.

Outre la forme du contour C des trous traversants 6, tout autre agencement de trous traversants 6 pourrait être prévu. En particulier, les trous traversants 6 pourraient être alignés selon l'une ou l'autre des première et deuxième directions ou autrement répartis.

Par ailleurs, le pansement interface 1 pourrait comprendre une armature supportant la composition élastomérique.

### Elastomère

La composition peut comprendre au moins un copolymère tribloc du type ABA.

Les copolymères séquencés peuvent être des copolymères triblocs du type ABA comportant deux blocs terminaux thermoplastiques A styrène et une séquence centrale élastomère B qui est une oléfine saturée. Les séquences B d'oléfines saturées sont, par exemple, des séquences éthylène-butylène, éthylène-propylène ou éthylène-éthylène-propylène.

Par souci de simplicité, dans la présente description, les blocs polymériques constituant les copolymères précités sont désignés par la nature de leurs unités récurrente. Ainsi, l'expression « bloc » ou « séquence styrène A » désigne une séquence poly(styrène) et l'expression « bloc » ou « séquence oléfine saturée » désigne une séquence poly(oléfine saturée).

Selon un mode de réalisation, la composition comprend un mélange de deux copolymères, ledit mélange comprenant au moins un copolymère qui présente une viscosité comprise entre 0,01 et 1 Pa.s mesurée dans une solution à 5 % masse/masse dans le toluène et au moins un copolymère présentant une viscosité comprise entre 0,01 et 0,5 Pa.s mesurée dans une solution à 15% (masse/masse) dans le toluène.

Les copolymères triblocs à séquence centrale saturée sont bien connus de l'homme de l'art et sont, par exemple, commercialisés :
- par la société KRATON sous la dénomination KRATON® G, et en particulier les grades KRATON® G1651, KRATON® G1654, KRATON® G 1657, KRATON® G1652 ou KRATON® G1650 et par la société KURARAY sous les dénominations SEPTON® et en particulier les grades 8006 ou 8004 pour les copolymères séquencés poly (styrène-éthylène-butylène-styrène) (en abrégé SEBS);
- par la société KURARAY sous la dénomination SEPTON® pour les copolymères séquencés poly (styrène-éthylène-propylène-styrène) (en abrégé SEPS) et en particulier les grades 2005, 2006 ou 2063 et pour les polymères séquencés poly (styrène-éthylène-éthylène-propylène-styrène) (en abrégé SEEPS) et en particulier les grades 4033, 4044, 4055, 4077 ou 4099.

Parmi les copolymères qui présentent une viscosité comprise entre 0,01 et 1 Pa.seconde mesurée dans une solution à 5% (masse/masse) dans le toluène, on peut citer les copolymères commercialisé par la société KRATON sous les grades KRATON® G 1651 et KRATON® G 1654 et les copolymères commercialisés par la société KURARAY sous les grades SEPTON® 2005, 2006, 8006, 4055, 4077, 4044 ou 4099.

Parmi les copolymères qui présentent une viscosité comprise entre 0,01 et 0,5 Pa.seconde mesurée dans une solution à 15% (masse/masse) dans le toluène on peut citer les copolymères commercialisés par la société KRATON sous les grades KRATON® G 1650, KRATON® G 1657 et KRATON® G 1652 et les copolymères commercialisés par la société KURARAY sous les grades SEPTON® 2063 ou 4033.

Ces viscosités sont mesurées à 30°C à l'aide d'un viscosimètre Brookfield modèle LVI dans une solution dans le toluène à 5 % ou 15 % masse/masse en fonction du poids moléculaire du copolymère.

On préférera les copolymères triblocs SEBS, SEPS ou SEEPS ayant une teneur en styrène comprise entre 25 % et 45 % en poids par rapport au poids dudit copolymère SEBS, SEPS ou SEEPS.

De façon générale, la quantité de copolymères dans la composition finale pourra être comprise entre 5 et 20 % en poids, de préférence entre 7 et 15 % en poids, rapportée au poids total de la composition.

On préférera tout particulièrement l'utilisation de deux copolymères séquencés SEBS, et en particulier l'association des copolymères KRATON® G 1654 et KRATON® G 1650 dans laquelle le KRATON® G 1654 est présent en une quantité de 5 à 10% en poids, rapportée au poids total de la composition et le KRATON® G 1650 est présent en une quantité de 2 à 5% en poids, rapportée au poids total de la composition.

De préférence, ce mélange de deux copolymères comprendra donc au moins 5 à 10% en poids d'un copolymère qui présente une viscosité comprise entre 0,01 et 1 Pa.seconde mesurée dans une solution à 5 % masse/masse dans le toluène et au moins 2 à 5% d'un copolymère présentant une viscosité comprise entre 0,01 et 0,5 Pa.seconde mesurée dans une solution à 15% (masse/masse) dans le toluène, rapporté au poids total de la composition.

### Les résines

La matrice élastomérique peut également contenir une résine. Les résines utilisées sont celles habituellement utilisées dans ce type de matrice.

Parmi ces résines, on citera à titre d'exemple les résines généralement employées par l'homme de l'art telles que les résines polyterpènes ou terpènes modifiées, les résines de colophane hydrogénée, les résines de colophane polymérisée, les résines d'esters de colophane, les résines hydrocarbonnées, les mélanges de résines aromatiques et aliphatiques etc... On peut citer à titre d'exemple une résine de synthèse formée de copolymères en C5/C9 commercialisée par la société CRAY VALLEY sous la dénomination WINGTACK 86.

Parmi les résines d'hydrocarbure(s) hydrogéné(s), on citera les résines commercialisées par exemple par la société ARAKAWA sous la dénomination ARKON®.

La composition peut également comprendre au moins une résine tackifiante pour leur conférer un caractère adhésif facilitant leur positionnement sur la plaie.

Les résines tackifiantes pouvant optionnellement être mises en œuvre dans la composition sont choisies notamment parmi les polyisobutylènes à bas poids moléculaire. De façon générale, on préfère l'utilisation de résines hydrogénées telles que les résines Escorez® de la série 5000, et encore plus préférentiellement, la résine Escorez 5380®.

Les résines mises en œuvre préférentiellement dans la composition sont des résines hydrocarbonées aromatiques, c'est-à-dire à base de monomères aromatiques uniquement. Elles se distinguent des résines aliphatiques, à base de monomères aliphatiques uniquement, ou des résines aliphatiques/aromatiques à base de monomères aliphatiques et aromatiques. Sans vouloir être lié par une quelconque théorie, il semble que ces résines présentent une bonne solubilité dans le bloc A des copolymères ABA et renforcent ce bloc styrène, ce qui améliore la cohésion de la matrice élastomérique finale obtenue.

En particulier, le monomère aromatique est l'alpha-méthyl styrène. Ainsi, selon un mode de réalisation, la résine hydrocarbonée aromatique est choisie parmi les résines d'homopolymères et copolymères d'alpha-méthyl-styrène.

Parmi les résines aromatiques testées, un certain nombre d'entre elles ne donnaient pas entière satisfaction. En effet, certains grades de résine, du fait de leur point de ramolissement élevé, nécessitent d'être chauffées à des températures élevées (au-delà de 140°C) pour réaliser la composition de l'invention. En travaillant à de telles températures, il existe un risque d'évaporation du plastifiant. Lorsque l'on ajoute des hydrocolloïdes (tels que la carboxymethylcellulose) ou des actifs dans la composition, ceux-ci risquent de se dégrader.

Ainsi, les résines mises en œuvre dans les compositions sont les résines de type alpha-méthyl styrène dont le point de ramollissement se situe entre 80 et 125°C, de préférence entre 90 et 110°C.

Le point de ramollissement est mesuré selon la norme ISO 4625 (méthode "Ring and Ball").

De préférence, la résine est une résine alpha-méthyl styrène présentant un point de ramollissement situé entre 95 et 105°C ou entre 115 et 125°C ou une résine poly(styrène-co-alpha-méthyl styrène) présentant un point de ramollissement entre 95°C et 115°C.

Les résines préférentielles ci-dessus sont bien connues de l'homme du métier et disponibles commercialement, par exemple vendues sous les dénominations commerciales suivantes :
- Sylvares SA 100 et Sylvares SA 120 d'Arizona Chemical : résines alpha-méthyl styrène présentant un point de ramollissement situé entre 95 et 105°C ou entre 115 et 125°C respectivement,
- la résine Cleartack W90 ou Norsolène W90 de Cray Valley : résine poly(styrene-co-alpha-methylstyrene) présentant un point de ramollissement entre 85 et 95°C,
- les résines Kristalex 3100LV, Kristalex F100, Kristalex 3105SD et Kristalex F115 d'Eastman : résines poly(styrene-co-alpha-methylstyrene) présentant un point de ramollissement de 100°C, ou entre 96 et 104°C ou de 105°C, ou entre 114 et 120°C respectivement.

La résine est de préférence présente en une quantité de 5 à 20%, de préférence 5 à 15% en poids, rapportée au poids total de la composition.

Afin de réaliser des pansements interface, le mélange de copolymères et la résine présents dans la composition sont associés à un (ou plusieurs) composé(s) plastifiants.

Les plastifiants susceptibles d'être utilisés sont bien connus et destinés à améliorer les propriétés d'étirement, de souplesse, d'extrudabilité ou de mise en œuvre des copolymères. On pourra utiliser à cet effet un ou plusieurs plastifiants si nécessaire.

D'une façon générale, en tant que plastifiants, on préférera des composés liquides, compatibles avec la séquence centrale oléfine saturée des copolymères séquencés précités.

Parmi les composés plastifiants susceptibles d'être utilisés à cet effet, on citera en particulier les huiles minérales plastifiantes.

Alternativement, on peut aussi utiliser des produits de synthèse à base de mélanges liquides d'hydrocarbures saturés comme par exemple les produits commercialisés par la société TOTAL sous la dénomination GEMSEAL® et en particulier le produit GEMSEAL® 60 qui est un mélange isoparaffinique issu d'une coupe pétrolière totalement hydrogénée.

On utilisera de préférence des huiles plastifiantes et en particulier des huiles minérales formées de composés de nature paraffinique ou naphténique, ou de leurs mélanges, dans des proportions variables.

Des huiles minérales plastifiantes particulièrement préférées sont formées de mélanges de composés de nature paraffinique et naphténique, et en particulier de tels mélanges dans lesquels la proportion de composés de nature paraffinique est majoritaire.

Parmi les huiles plastifiantes convenant particulièrement, on peut citer les produits commercialisés par la société SHELL sous les dénominations ONDINA® et en particulier ONDINA® 919 ou l'huile commercialisée par la société PETRO CANADA sous la référence PURETOL® 9D ou l'huile BLANDOL commercialisée par Sonneborn ou encore l'huile Pionier 2076P commercialisé par Hansen & Rosenthal.

Outre des huiles, le plastifiant peut comprendre de la vaseline. La vaseline mise en œuvre dans la composition est une vaseline conforme à la Pharmacopée Française disponible commercialement.

La vaseline est présente en une quantité de 1 à 30%, de préférence 5 à 25% en poids, rapportée au poids total de la composition.

Le plastifiant est présent en une quantité de 50 à 80%, de préférence 60 à 70% en poids, rapportée au poids total de la composition.

De préférence, le plastifiant est constitué d'un mélange d'huile minérale et de vaseline, l'huile minérale étant présente en une quantité allant de 45 à 60% en poids rapporté au poids total de la composition, la vaseline étant présente en une quantité allant de 5 à 20% en poids rapporté au poids total de la composition.

### Actif

La composition peut également comprendre au moins un actif permettant d'induire ou d'accélérer la cicatrisation ou pouvant avoir un rôle favorable dans le traitement d'une plaie.

Parmi ces substances actives, on peut citer, en particulier, à titre d'exemples :
- les agents favorisant la cicatrisation tels que le rétinol, la vitamine A, la vitamine E, la N-Acétyl Hydroxyproline, les extraits de Centella Asiatica, la papaïne, la silicone, les huiles essentielles de thym, niaouli, romarin, sauge, l'acide hyaluronique, le sucrose octasulfate de potassium, le sucralfate, l'allantoïne, la metformine ;
- les agents antibactériens tels que les sels ou complexes d'argent (tels les sulfates d'argent, les nitrates d'argent, les sulfamides d'argent ou encore les zéolites à base d'argent), les sels de zinc ou de cuivre, le métronidazole, la néomycine, les pénicillines, l'acide clavulanique, les tétracyclines, la mynocycline, la chlorotétracycline, les aminoglycosides, l'amikacine, la gentamicine, les probiotiques ;
- les antiseptiques tels que la chlorhexidine, le trichlosan, le biguanide, l'hexamidine, le thymol, le lugol, la povidone iodée, le chlorure de benzalkonium et de benzethonium ;
- les anti-douleurs tels que le paracétamol, la codéine, le dextropropoxyphène, le tramadol, la morphine et ses dérivés, les corticoïdes et leurs dérivés ;
- les anesthésiques locaux tels que la lidocaïne, la benzocaïne, la dibucaïne, le chlorhydrate de pramoxine, la bupivacaïne, la mépivacaïne, la prilocaïne, l'étidocaïne ;
- les anti-inflammatoires comme les anti-inflammatoires non stéroïdiens (AINS), l'aspirine ou acide acétylsalicylique, l'ibuprofène, le kétoprofène, le flurbiprofène, le diclofenac, l'acéclophénac, le kétorolac, le méloxicam, le piroxicam, le ténoxicam, le naproxène, l'indométacine, le naproxcinod, le nimésulid, le célécoxib, l'étoricoxib, le parécoxib, le rofécoxib, le valdécoxib, la phénylbutazone, l'acide niflumique, l'acide méfénamique;
Bien entendu, la composition peut aussi comprendre un ou plusieurs autres composés connus pour leur action dans la phase de détersion comme par exemple
- des enzymes ;
- l'urée.

De préférence, l'agent favorisant la cicatrisation est choisi parmi les agents favorisant la cicatrisation le rétinol, la vitamine A, la vitamine E, la N-Acétyl Hydroxyproline, les extraits de Centella Asiatica, la papaïne, la silicone, les huiles essentielles de thym, niaouli, romarin, sauge, l'acide hyaluronique, le sucrose octasulfate de potassium, le sucralfate, l'allantoïne, la metformine, et de préférence, l'agent favorisant la cicatrisation est la metformine.

La composition comprend 0,1 à 15% d'actifs, de préférence 1 à 10% en poids, rapporté au poids total de la composition.

### Hydrocolloïde

Dans le cadre de la réalisation de pansements interface, avec support ou avec armature pour la cicatrisation des plaies, la composition peut comprendre des particules hydrophiles d'un hydrocolloïde (ou particules d'hydrocolloïde).

Ces particules permettent en effet le retrait indolore d'un pansement interface et le maintien d'un milieu humide au niveau de la plaie afin de favoriser la cicatrisation.

A cet effet, une quantité faible de particules hydrophiles d'un hydrocolloïde est ainsi soit disposée en surface de la matrice élastomérique une fois celle-ci formée soit, de préférence, dispersée de façon homogène au sein de la composition.

Par « hydrocolloïde » ou « particules d'hydrocolloïde », on entend désigner ici tout composé habituellement utilisé par l'homme de l'art pour son aptitude à absorber les liquides aqueux tels que l'eau, le sérum physiologique ou les exsudats d'une plaie.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium, ainsi que les polymères synthétiques à base de sels de l'acide acrylique, connus sous l'appellation "superabsorbants", comme par exemple les produits commercialisés par la société CIBA Specialty Chemicals sous la dénomination SALCARE® SC91 ainsi que les mélanges de ces composés.

Certains de ces superabsorbants qualifiés de « microcolloïdes » car ils présentent une taille de particules inférieure à 10 micromètres peuvent bien entendu être également utilisés.

Les hydrocolloïdes préférés sont les sels de métal alcalin de la carboxyméthylcellulose, et en particulier la carboxyméthylcellulose de sodium (CMC).

La taille des particules d'hydrocolloïde est généralement comprise entre 50 et 100 microns, avantageusement de l'ordre de 80 microns.

D'une façon générale, la quantité de particules d'hydrocolloïde incorporées dans la composition sera avantageusement inférieure ou égale à 25 % en poids, avantageusement de l'ordre de 2 à 20 % en poids, de préférence de 5 à 18 % en poids, de préférence encore de 10 à 15 % en poids, rapportée au poids total de ladite composition.

Si les particules d'hydrocolloïde sont disposées en surface de la matrice élastomérique un fois celle-ci formée, leur quantité sera de préférence de l'ordre de 1 à 10 % et plus particulièrement de 2 à 5 % en poids, rapportée au poids total de ladite matrice élastomérique.

Le choix d'une quantité de particules d'hydrocolloïde comprise dans ces plages de valeurs est importante pour la réalisation d'un pansement interface, et en particulier un pansement interface autoporté aéré, afin d'éviter que la gélification de la composition n'entraine la fermeture des trous traversants lors de l'absorption des exsudats.

### Antioxydants

La composition peut également comprendre des agents antioxydants.

Par « agents antioxydants », on entend désigner ici les composés couramment employés par l'homme de l'art pour assurer la stabilité des composés entrant dans la formulation des compositions, en particulier vis-à-vis de l'oxygène, la chaleur, l'ozone ou les rayonnements ultra-violets.

Comme exemples d'agents antioxydants appropriés, on peut citer notamment les antioxydants phénoliques comme en particulier les produits commercialisés par la société BASF sous les dénominations IRGANOX® 1010, IRGANOX® 565, IRGANOX® 1076.

D'une façon générale, ces agents antioxydants pourront être utilisés seuls ou en association en une quantité de l'ordre de 0,05 à 1 % en poids, de préférence de 0,05 à 0,2 % en poids, rapportée au poids total de la composition.

On préférera l'utilisation du produit IRGANOX® 1010 en une quantité comprise entre 0,05 et 0 ,2 % en poids, rapportée au poids total de la composition.

### Adjuvant

A titre d'adjuvants susceptibles d'être utilisés dans la composition, on peut citer des composés connus pour favoriser le relargage des agents actifs, comme par exemple les produits Montanox® 80 ou Sepinov® EMT 10 (copolymère du sel de l'acide 2-méthyl-2[(1-oxo-2-propényl)amino]-1-propanesulfonique et de l'ester 2-hydroxyéthyle de l'acide propénoïque ou du mélange de 2-octyl-1-dodecanol, de D-xylopyranoside, de 2-octyldodécyl et de polyéthylèneglycol 30 dipolyhydroxystéarate) qui sont couramment utilisés dans les produits URGOTUL® qui incorporent des agents actifs.

Ces adjuvants pourront être utilisés en une quantité de l'ordre de 0,01 à 10% en poids, de préférence 0,05 à 5% en poids, par rapport au poids total de la matrice élastomérique.

Bien évidemment les modes de réalisation particuliers qui viennent d'être décrits peuvent être mis en œuvre séparément ou selon l'une quelconque de leurs combinaisons.

La composition selon l'invention permet, en particulier, de réaliser un pansement interface autoporté ou un pansement interface présentant une armature ou un support.

Dans le cadre de la réalisation d'un pansement interface, on préférera l'utilisation d'une composition qui comprend des composés (copolymères, huile minérale, vaseline, antioxydant et hydrocolloïdes) de même nature que, ou identiques à, ceux utilisés dans le produit URGOTUL®.

### Matrice élastomérique

Afin de réaliser un pansement interface, la composition sera formée en couche mince, avec des trous traversants, disposés de préférence de façon répartie dans ladite couche pour former une matrice élastomérique.

### Pansement

Le pansement interface autoporté comprend une matrice élastomérique se présentant sous la forme d'une couche mince possédant des trous traversants pour laisser passer les exsudats, obtenue à partir d'une composition comprenant :
- 5 à 20% d'au moins un copolymère tribloc du type styrène - oléfine saturée - styrène,
- 50 à 80% en poids d'au moins un plastifiant,
- 5 à 20% d'au moins une résine
les pourcentages étant rapportés au poids total de la composition.

Selon un mode préféré de réalisation, le pansement interface autoporté comprend une matrice élastomérique se présentant sous la forme d'une couche mince possédant des trous traversants pour laisser passer les exsudats, obtenue à partir d'une composition comprenant :
- 5 à 20% d'au moins un copolymère tribloc du type styrène - oléfine saturée - styrène,
- 50 à 80% en poids d'au moins un plastifiant,
- 5 à 20% d'au moins une résine de type alpha-méthyl styrène dont le point de ramollissement se situe entre 80 et 125°C, de préférence entre 90 et 110°C,
les pourcentages étant rapportés au poids total de la composition.

De préférence, le pansement interface n'adhère pas aux gants en latex. Pour ce faire, la composition peut, de préférence, comprendre :
- pour 100 parties en poids d'un mélange P de 2 copolymères tribloc spécifiques du type styrène - oléfine saturée - styrène, un premier qui présente une viscosité comprise entre 0,01 et 1 Pa.s telle que mesurée dans une solution à 5 % (masse/masse) dans le toluène et un second qui présente une viscosité comprise entre 0 ,01 et 0,5 Pa.s telle que mesurée dans une solution à 15 % (masse/masse) dans le toluène ;
- de 300 à 1000 parties en poids d'un plastifiant H, de préférence une huile plastifiante ; et
- de 90 à 600 parties en poids de vaseline V ;
étant en outre précisé que :
- la quantité totale, représentée par P+H+V, de mélange d'élastomères, du plastifiant et de la vaseline est comprise entre 490 et 1700 parties en poids ;
- le rapport entre la quantité totale du mélange d'élastomères, du plastifiant et de la vaseline et la quantité de vaseline, représenté par P+H+V/V, est inférieur à 11;

ledit mélange de 2 copolymères comprend au moins 20 % en poids du premier copolymère,
la composition comprenant en outre de 5 à 20 % en poids d'une résine
les pourcentages étant rapportés au poids total de la composition.

Afin de protéger la composition de l'environnement extérieur, le pansement interface pourra être recouvert, de préférence sur chacune de ses faces, par un film protecteur provisoire qui sera retiré avant usage par l'utilisateur.

Afin de faciliter encore la manipulation du pansement interface, en particulier s'il est autoporté, on pourra substituer ces deux films protecteurs provisoires par un protecteur unique tel que décrit dans la demande de brevet WO 2008/145 884 ou dans la demande de brevet WO 2015/018720 dont la structure particulière facilite l'application du pansement sur la plaie.

Bien évidemment les modes de réalisation particuliers qui viennent d'être décrits peuvent être mis en œuvre séparément ou selon l'une quelconque de leurs combinaisons.

### Préparation des pansements interface autoportés

Des pansements interface selon des premier, deuxième, troisième, quatrième, cinquième et sixième exemples, ci-après également désignés respectivement par exemples 1, 2, 3, 4, 5, 6, sont réalisés en vue de réaliser des essais comparatifs.

La composition des exemples 1 à 6 a été élaborée à l'aide des constituants suivants dans les proportions, exprimées en pourcentage en poids, mentionnées dans le tableau 1.
Elastomères : copolymères séquencés de poly (styrène-éthylène-butylène-styrène) (en abrégé SEBS) :
- KRATON® G 1654 ES viscosité à 5% (masse/masse) dans le toluène : 0,02 Pa.s
- KRATON G 1651
- KRATON® G 1650 E viscosité à 15% (masse / masse) dans le toluène : 0,2 Pa.s
Plastifiant : huile minérale Pionier 2076P commercialisée par Hansen & Rosenthal
- vaseline : vaseline Codex® A commercialisée par la société AIGLON Antioxydant : IRGANOX® 1010 commercialisé par la société BASF
- hydrocolloïde: Carboxyméthylcellulose sodique CMC BLANOSE® 7H4XF commercialisée par la société ASHLAND,
Résine :
- Sylvares SA 100, résine alpha methyl styrene présentant un point de ramollissement situé entre 95 et 105°C, commercialisée par Arizona Chemical

### Fabrication de la composition

Dans un mélangeur vertical, on a introduit successivement à une température de consigne de 90°C le plastifiant, l'hydrocolloïde et la vaseline et on a agité jusqu'à l'obtention d'un mélange homogène.

On a ensuite introduit le(s) copolymère(s) et l'antioxydant, augmenté la température de consigne à 150°C, puis on a agité jusqu'à l'obtention d'un mélange homogène. On a ajouté ensuite la(es) résine(s) le cas échéant.

On a ensuite laissé refroidir, puis on a vidangé le mélangeur.

Dans un deuxième temps, le mélange est réchauffé à 125°C puis déposé sur une plaque gravée, chauffée elle-même à 120°C. Le mélange ainsi déposé est arrasé à l'aide d'une râcle (également chauffée à 120°C) afin d'être réparti dans l'emprunte du moule.

La plaque gravée est ensuite refroidie jusqu'à une température de 40 à 50°C. Le mélange refroidi est alors retiré manuellement de la plaque gravée.

Pour l'exemple 5, le mélange est réchauffé à 120°C via une cuve chauffante. La matière est ensuite transportée grâce à des tuyaux chauffés à 90°C jusqu'à des têtes chauffantes équipées de buses de 0,5 mm maintenues à 98°C dont le diamètre de l'orifice est variable selon le besoin. L'ensemble tuyaux, têtes et buses est installé sur un portique XYZ, qui permet le dépôt de la matière sur les 3 dimensions. Le pansement interface autoporté est réalisé en deux temps : d'abord le contour extérieur, puis le dessin de l'intérieur du pansement (le parcours représente des marches décalées d'un incrément défini au préalable).

Pour l'exemple 6, le mélange est réchauffé à 130°C et enduit sur un tricot à fils tramés thermofixé, en fils de polyester, fabriqué par la société MDB TEXINOV sous la référence 601.

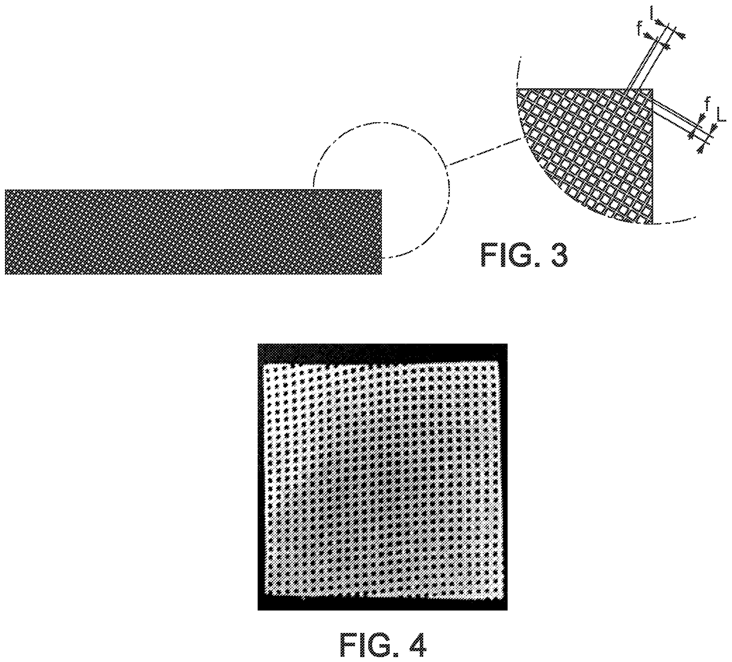

Sur les figures 3 à 5, les pansements interface selon les deuxième et troisième exemples sont conformes à des modes de réalisation de l'état de la technique. Dans ces pansements interface selon les deuxième et troisième exemples, les trous traversants sont alignés et leurs contours sont carrés identiques avec une seule et même orientation. Ces pansements interface diffèrent l'un de l'autre par leurs dimensions, à savoir leur longueur L et leur largeur 1.

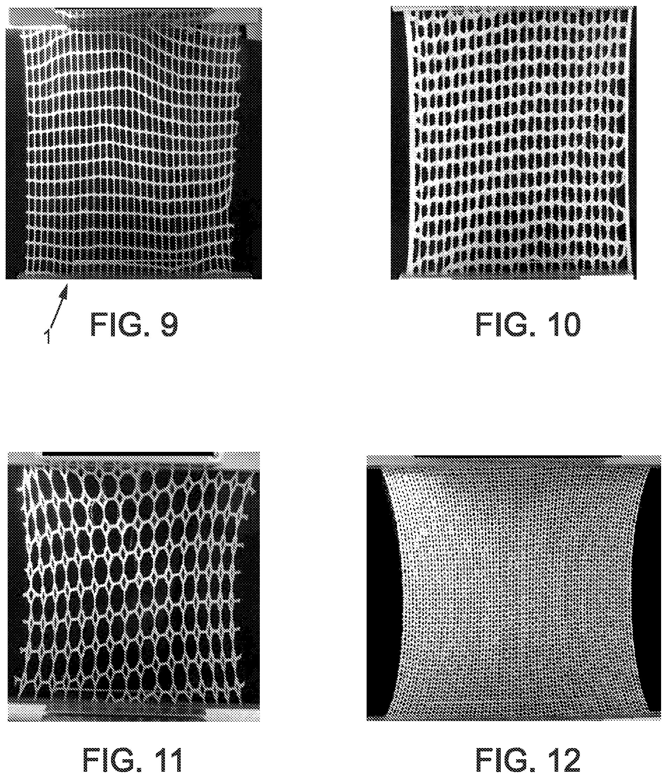

Le quatrième exemple de pansement interface visible sur la figure 11 comporte des trous traversants qui sont alignés et dont les contours sont en octaèdre identiques avec une seule et même orientation.

Le cinquième exemple de pansement interface visible sur la figure 10 est conforme à un mode de réalisation de l'invention et diffère du premier exemple en ce qu'il est obtenu par impression en trois dimensions (3D).

Le sixième exemple de pansement interface est conforme à un mode de réalisation de l'état de la technique comme les deuxième et troisième exemples dont il diffère par les dimensions.

Les principales caractéristiques des pansements interface selon des deuxième, troisième, quatrième, cinquième et sixième exemples sont données dans les tableaux 1, 2 et 3 ci-dessous.

**Tableau 1 :**

| | Masse des Ex.1, 2 et 3 | Masse des ex.4 et 5 | Masse de l'ex. 6 |
|---|---|---|---|
| *N° de masse interne* | *4488* | *3973* | |
| Kraton G1654 ES | 7.8 | 5.7 | |
| Kraton G 1650 E | 3.5 | 2.6 | |
| Kraton G 1651 | | | 4, 931 |
| Pionier 2076P | 53.5 | 61.5 | 74, 947 |
| Vaseline Codex A | 12 | 15 | 5, 000 |
| CMC Blanose 7H4XF | 13 | 15 | 14, 999 |
| Irganox 1010 | 0.2 | 0.2 | 0, 123 |
| Sylvares SA 100 | 10 | | |

**Tableau 2 :**

| | Motif | Dimensions trous traversant L x 1 (mm) | Largeur du fil f (mm) | Profondeur p ou épaisseur (µm) | Surface enduite (%) |
|---|---|---|---|---|---|
| Ex. 1 | Rectangles Tetris | 2.95*1.45 | 0.8 | 800 | 52.5 |
| Ex. 2 | Carrés | 2*2 | 0.8 | 800 | ≈50 |
| Ex. 3 | Carrés | 2*2 | 1.6 | 750 | 69 |
| Ex.4 | « Cabochon » | 1.95*1.95 | 0.8 | 500 | 53 |
| Ex. 5 | Rectangle | 3.0*2.0 | 1.3 | 1100 | 42 |
| Ex. 6 | carré | 0.8*0.8 | 0.4 | 260 | 40 |

**Tableau 3 :**

| | Motif | Epaisseur de la maquette (µm) | Grammage de la maquette (g/m²) |
|---|---|---|---|
| Ex. 1 | Rectangles Tetris | 755±60 | 390±12 |
| Ex. 2 | Carrés | 815±35 | 330±2 |
| Ex. 3 | Carrés | 700±35 | 400±13 |
| Ex. 4 | « Cabochon » | 350 | 129 |
| Ex. 5 | Rectangle | 1100 | 563 |
| Ex. 6 | Carrés | 260 | 158 |

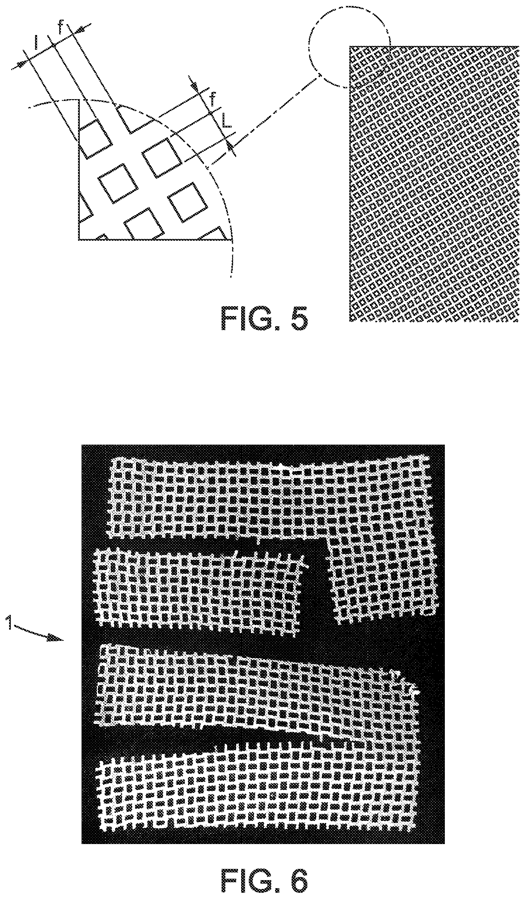

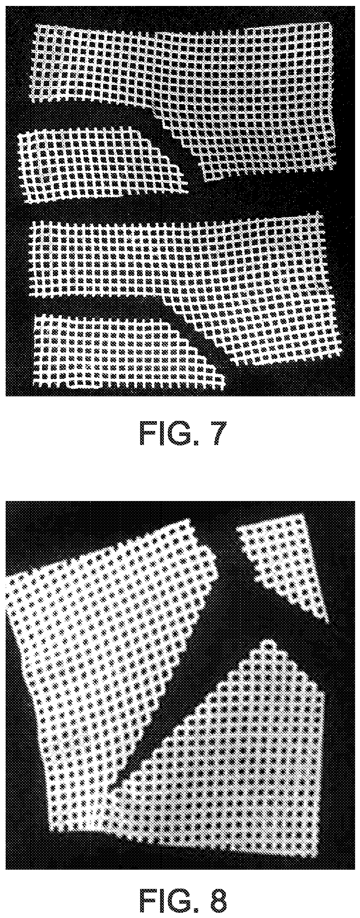

Sur les figures 6 à 8, les pansements interface selon les premier, deuxième et troisième exemples sont découpés manuellement. On constate que le pansement interface du premier exemple réalisé conformément à l'invention se découpe de manière linéaire à partir d'une amorce, ce qui n'est pas le cas des pansements interface selon les deuxième et troisième exemples conforme à des modes de réalisation de l'état de la technique.

Sur les figures 9 à 12, au moyen d'une machine de traction à vitesse constante d'allongement, les pansements interface selon les exemples premier (figure 9), cinquième (figure 10), quatrième (figure 11) et sixième (figure 12), de largeur 1 10 cm, sont soumis à une traction selon une direction d'étirement parallèle à leur longueur, à une vitesse de 100 mm/min, avec une distance entre les mors de 5 cm, jusqu'à un allongement d'environ 70% de leurs allongements à la rupture. Les pansements interface sont maintenus à cet allongement pendant quelques secondes afin d'observer la géométrie de leurs bords latéraux.

Lorsqu'ils sont soumis à une traction, les pansements interface des premier et cinquième exemples (figures 9 et 10), conforme à l'invention, qu'ils soient obtenus par moulage ou par impression 3D, restent droits au niveau de leurs bords latéraux, contrairement aux pansements interface des quatrième (figure 11) et sixième (figure 12) exemples dont les bords latéraux sont déformés pour devenir concaves.

## Revendications

1. Pansement interface (1) comprenant une matrice élastomérique (5), la matrice élastomérique (5) étant pourvue d'une pluralité de trous traversants (6), chaque trou traversant (6) ayant un contour (C) autour d'un axe central (A),
le pansement interface (1) étant **caractérisé en ce qu'**au moins une partie de la pluralité de trous traversants (1) est agencée de manière à former au moins un motif dans lequel chaque trou traversant (6) est adjacent à au moins un autre trou traversant (6), les trous traversants (6) adjacents ayant des contours (C) dont des images par une translation dans un plan perpendiculaire aux axes centraux (A) pour confondre des images desdits axes centraux (A) par ladite translation ne se superposent pas.

2. Pansement interface (1) selon la revendication 1, dans lequel la matrice élastomérique (5) constituée d'une composition élastomérique, les trous traversants (6) adjacents du motif étant séparés l'un de l'autre par un fil (7) de composition élastomérique.

3. Pansement interface (1) selon la revendication 2, constitué de la seule matrice élastomérique (5) et dépourvu d'armature supportant la composition élastomérique, de manière à être autoporté.

4. Pansement interface (1) selon l'une quelconque des revendications 1 à 3, dans lequel le motif comprend des trous traversants (6) adjacents ayant des contours (C) identiques décalés l'un par rapport à l'autre angulairement autour des axes centraux (A) respectifs.

5. Pansement interface (1) selon la revendication 4, dans lequel le motif comprend des trous traversants (6) adjacents ayant des contours (C) dépourvus chacun de symétrie de rotation autour de l'axe central (A).

6. Pansement interface (1) selon l'une quelconque des revendications 4 et 5, dans lequel le motif comprend des trous traversants (6) adjacents ayant des contours (C) présentant chacun une symétrie de rotation autour de l'axe central (A) par rotation d'angle 2π/n radians, n entier, les contours (C) des trous traversants (6) adjacents du motif étant décalés l'un par rapport à l'autre angulairement autour des axes centraux (A) respectifs avec des angles différents de 2π/n radians.

7. Pansement interface (1) selon l'une quelconque des revendications 1 à 6, dans lequel le motif comprend des trous traversants (6) adjacents présentant des contours (C) rectangulaires.

8. Pansement interface (1) selon la revendication 7, dans lequel les contours (C) rectangulaires des trous traversants (6) adjacents sont agencés orthogonalement l'un par rapport à l'autre.

9. Pansement interface (1) selon l'une quelconque des revendications 1 à 8, dans lequel le motif comprend un réseau de trous traversants (1) adjacents dont les axes centraux (A) sont alignés selon au moins une première direction (X).

10. Pansement interface (1) selon la revendication 9, dans lequel les axes centraux (A) des trous traversants (6) adjacents du réseau sont alignés selon une deuxième direction (Y) perpendiculaire à la première direction (X).

## Patentansprüche

1. Grenzflächenverband (1) mit einer elastomeren Matrix (5), wobei die elastomere Matrix (5) mit einer Vielzahl von Durchgangslöchern (6) versehen ist, jedes Durchgangsloch (6) eine Kontur (C) um eine zentrale Achse (A) aufweist,
wobei der Grenzflächenverband(1) **dadurch gekennzeichnet ist, dass** mindestens ein Teil der Vielzahl von Durchgangslöchern (6) so angeordnet ist, dass es mindestens ein Muster bildet, in dem jedes Durchgangsloch (6) an mindestens ein anderes Durchgangsloch (6) angrenzt wobei die benachbarten Durchgangslöcher (6) Konturen (C) aufweisen, deren Bilder durch eine Verschiebung in einer Ebene senkrecht zu den Mittelachsen (A), um Bilder der Mittelachsen (A) durch die Verschiebung zu verschmelzen, nicht überlappen.

2. Grenzflächenverband (1) nach Anspruch 1, wobei die Elastomermatrix (5) aus einer Elastomerzusammensetzung besteht, wobei die benachbarten Durchgangslöcher (6) des Musters durch einen Faden (7) aus Elastomerzusammensetzung voneinander getrennt sind.

3. Grenzflächenverband (1) nach Anspruch 2, bestehend aus der elastomeren Matrix (5) allein und ohne Verstärkung, die die elastomere Zusammensetzung trägt, so dass sie selbsttragend ist.

4. Grenzflächenverband (1) nach einem der Ansprüche 1 bis 3, wobei das Muster benachbarte Durchgangslöcher (6) mit identischen Konturen (C) umfasst, die um jeweilige Mittelachsen (A) winkelversetzt zueinander sind.

5. Grenzflächenverband (1) nach Anspruch 4, wobei das Muster besteht aus benachbarten Durchgangslöchern (6) mit Konturen (C), denen jeweils die Rotationssymmetrie um die Mittelachse (A) fehlt.

6. Grenzflächenverband (1) nach einem der Ansprüche 4 und 5, wobei das Muster benachbarte Durchgangslöcher (6) umfasst, die Konturen (C) aufweisen, die jeweils um einen Winkel von 2π/n Radianten rotationssymmetrisch um die zentrale Achse (A) sind, wobei n eine ganze Zahl ist, wobei die Konturen (C) der benachbarten Durchgangslöcher (6) des Musters um die jeweiligen zentralen Achsen (A) um Winkel, die sich von 2π/n Radianten unterscheiden, winkelmäßig gegeneinander verschoben sind.

7. Grenzflächenverband (1) nach einem der Ansprüche 1 bis 6, wobei das Muster benachbarte Durchgangslöcher (6) mit rechteckigen Konturen (C) umfasst.

8. Grenzflächenverband (1) nach Anspruch 7, wobei die Rechteckkonturen (C) benachbarter Durchgangslöcher (6) orthogonal zueinander angeordnet sind.

9. Grenzflächenverband (1) nach einem der Ansprüche 1 bis 8, wobei das Muster eine Anordnung von benachbarten Durchgangslöchern (1) umfasst, deren Mittelachsen (A) entlang mindestens einer ersten Richtung (X) ausgerichtet sind.

10. Grenzflächenverband (1) nach Anspruch 9, wobei die Mittelachsen (A) von benachbarten Durchgangslöchern (6) der Anordnung entlang einer zweiten Richtung (Y) senkrecht zur ersten Richtung (X) ausgerichtet sind.

## Claims

1. An interface dressing (1) comprising an elastomeric matrix (5), the elastomeric matrix (5) being provided with a plurality of through-holes (6), each through-hole (6) having a contour (C) about a central axis (A),
the interface dressing (1) being **characterized in that** at least one part of the plurality of through-holes (1) is arranged so as to form at least one pattern wherein each through-hole (6) is adjacent to at least one other through-hole (6), the adjacent through-holes (6) having contours (C), images of which, when moved translationally in a plane perpendicular to the central axes (A) in order to merge images of said central axes (A) by means of said translational movement, do not become superimposed.

2. The interface dressing (1) as claimed in claim 1, wherein the elastomeric matrix (5) consists of an elastomeric composition, the adjacent through-holes (6) of the pattern being separated from one another by a yarn (7) of elastomeric composition.

3. The interface dressing (1) as claimed in claim 2, consisting solely of the elastomeric matrix (5) and devoid of framework supporting the elastomeric composition, so as to be self-supported.

4. The interface dressing (1) as claimed in any one of claims 1 to 3, wherein the pattern comprises adjacent through-holes (6) which have identical contours (C) angularly offset from one another about the respective central axes (A).

5. The interface dressing (1) as claimed in claim 4, wherein the pattern comprises adjacent through-holes (6) which have contours (C) that are each devoid of rotational symmetry about the central axis (A).

6. The interface dressing (1) as claimed in either one of claims 4 and 5, wherein the pattern comprises adjacent through-holes (6) which have contours (C) that each exhibit a rotational symmetry about the central axis (A) with respect to rotation through an angle 2π/n radians, n being an integer, the contours (C) of the adjacent through-holes (6) of the pattern being angularly offset from one another about the respective central axes (A) with angles different than 2π/n radians.

7. The interface dressing (1) as claimed in any one of claims 1 to 6, wherein the pattern comprises adjacent through-holes (6) which have rectangular contours (C).

8. The interface dressing (1) as claimed in claim 7, wherein the rectangular contours (C) of the adjacent through-holes (6) are arranged orthogonally relative to one another.

9. The interface dressing (1) as claimed in any one of claims 1 to 8, wherein the pattern comprises a network of adjacent through-holes (1), the central axes (A) of which are aligned in at least a first direction (X).

10. The interface dressing (1) as claimed in claim 9, wherein the central axes (A) of the adjacent through-holes (6) of the network are aligned in a second direction (Y) perpendicular to the first direction (X).
